# EUROPEAN PATENT APPLICATION

(11) **EP 0 669 132 A1**
(43) Date of publication of application: **30.08.1995**
(21) Application number: 94200423.5
(22) Date of filing: 23.02.1994
(51) Int. Cl.: A61K 31/62

(54) **Pharmaceutical composition of vitamin E and acetylsalicylate for treatment and prevention of atherosclerosis**

(71) Applicant: van der Kraaij, Antonius Marinus Maria, NL-3069 XT Rotterdam (NL); van Iwaarden, Matthijs Jan, NL-3062 WN Rotterdam (NL); Liqui Lung, Arthur Frederick Leonard, NL-3068 KS Rotterdam (NL); Smith, Mike Stephan, NL-7315 AV Apeldoorn (NL)
(72) Inventor: van der Kraaij, Antonius Marinus Maria, NL-3069 XT Rotterdam (NL); van Iwaarden, Matthijs Jan, NL-3062 WN Rotterdam (NL); Liqui Lung, Arthur Frederick Leonard, NL-3068 KS Rotterdam (NL); Smith, Mike Stephan, NL-7315 AV Apeldoorn (NL)

(57) **Abstract**

The current invention involves a new treatment for human atherosclerosis. It concerns primary as well as secondary prevention of atherosclerosis by a pharmaceutical composition of vitamin E and acetylsalicylate. The development of atherosclerosis is suppressed at three different levels using this particular composition : 1) reduction of oxidation of LDL, in vivo, by vitamin E, 2) stabilization of endothelial cellmembranes by vitamin E making these cells less vulnerable to oxidation and 3) the anti-platelet action of acetylsalicylate which prevents platelet aggregation, thrombus formation and cytokines release which decreases inflammatory reactions and smooth-muscle-cell proliferation. An accidental advantage of the composition is the relatively small amount of acetylsalicylate that is needed when combined with vitamin E. This is quite important in view of the dose-dependent side-effects of acetylsalicylate. For these reasons, the composition can be expected to be effective and safe. It can be used widely against atherosclerosis and should be considered an additional weapon against atherosclerosis along with the reduction of known riskfactors such as hypertension, smoking, hypercholesterolemia and diabetes mellitus. The composition is designed to treat and/or prevent diseases arising from atherosclerosis such as angina pectoris, myocardial infarction, transient ischemic attacks, stroke and claudicatio intermittens.

## Description

The current invention involves a new treatment for human atherosclerosis. It concerns primary as well as secondary prevention of atherosclerosis by a pharmaceutical composition of vitamin E and acetylsalicylate.

With "vitamin E" is meant all stereoisomeric forms of alpha-tocopherol, beta-tocopherol, gamma-tocopherol, delta-tocopherol, alpha-tocotrienol, beta-tocotrienol, gamma-tocotrienol and delta-tocotrienol together with the acetate- and succinate-esters of these compounds. With "acetylsalicylate" is meant acetylsalicylic acid (C9 Hg 04) and all salts of acetylsalicylic acid such as sodium acetylsalicylate and calcium acetylsalicylate. With "pharmaceutical composition" is meant every orally active dosage form containing up to 500 mg of the active compound "acetylsalicylate" as well as up to 2000 IU of the active compound "vitamin E" (one IU is equivalent to one mg dl-alpha-tocopheryl acetate). At present, both vitamin E (1,2) and acetylsalicylate (3,4) are separately used as a drug. Vitamin E is a fat-soluble vitamin found predominantly in vegetable oils, eggs, cereals, liver and nuts. It acts as an antioxidant protecting polyunsaturated fatty acids, lipids and lipid-soluble substances from oxidation. In case of deficiency of vitamin E, as e.g. in cystic fibrosis or severe mal- absorption, hemolytic anemia develops due to per- oxidation of phospholipids in cellular membranes. This pathological condition can be successfully treated with vitamin E (5).

Acetylsalicylate, on the other hand, is a potent anti-inflammatory drug as it inhibits the biosynthesis of prostaglandins by inactivating the cyclooxygenase activity of prostaglandin-synthetase (6). Since prostaglandins enhance inflammatory reactions, acetylsalicylate is a potent anti-inflammatory drug. Besides this anti-inflammatory action, acetylsalicylate has strong anti-thrombotic effects as it blocks the synthesis of thromboxane A₂ in platelets by the same mechanism. Since thromboxane A₂ is responsible for platelet aggregation, acetylsalicylate is widely used to prevent thromboembolic complications in various diseases (7).

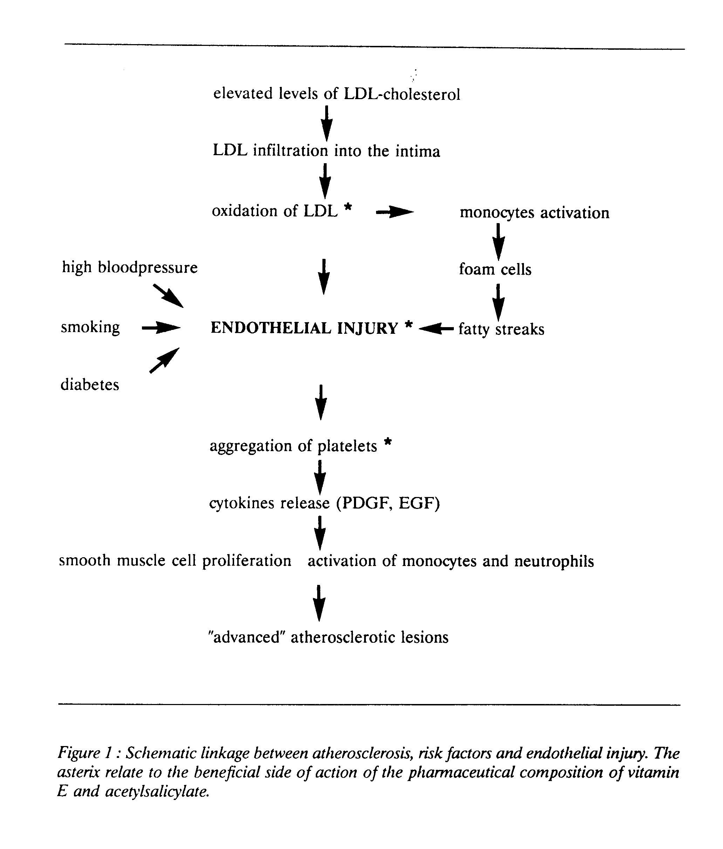

Today, atherosclerosis is known to be strongly associated with chronically elevated levels of plasma cholesterol, particularly cholesterol stored in low density lipoproteins (LDL). LDL itself is not atherogenic in contrast to its oxidized form. For this reason, oxidized LDL is held responsible for atherogenesis in contrast to LDL itself (8). During the modification of LDL to its oxidized form, extensive conversion of LDL-lecithin to LDL-lysolecithin takes place together with the formation of short-chain aldehydes such as malondialdehyde and 4-hydrox- ynonenal. All these substances are highly cytotoxic and able to initiate endothelial injury (fig.1). Moreover, these aldehydes act as chemotactic factors for neutrophils and monocytes augmenting inflammatory reactions. There is strong evidence to show that oxidized LDL is generated, in vivo, since oxidized LDL is largely present in atherosclerotic plaques and specific antibodies against oxidized LDL are found in human plasma. As oxidized LDL is cytotoxic it is thought to be responsible for endothelial injury in atherosclerosis. In addition, oxidized LDL is a potent chemo-attractant responsible for the migration of monocytes into the intima. Activated monocytes in the intima rapidly take up the oxidized LDL, thereby changing into foam cells and enhancing endothelial injury. In response to endothelial injury platelets adhere to the endothelium and release several cytokines such as platelet- derived-growth-factor (PDGF) and epidermal- growth-factor (EGF). These growth-factors further induce inflammatory reactions as well as smooth muscle cell proliferation forming "advanced lesions" which is a final step in atherogenesis (9).

The present invention relates to a pharmaceutical composition possessing remarkable anti-atherosclerotic effectiveness. The anti-sclerotic effect is induced by:
1) Reduction of oxidation of LDL, in vivo, by vitamin E.
2) Stabilization of endothelial cellmembranes by vitamin E making these cells less vulnerable to oxidation.
3) The anti-platelet action of acetylsalicylate which prevents platelet aggregation, thrombus formation and cytokines release which decreases inflammatory reactions and smooth muscle cell proliferation (fig.1).

By combining the beneficial effects of both vitamin E and acetylsalicylate into one pharmaceutical dosage form a powerful new drug is created against atherosclerosis. The development of atherosclerosis is suppressed at three different levels using the described composition (see above and figure 1). An accidental advantage of the composition is the relatively small amount of acetylsalicylate that is needed in combination with vitamin E. This is quite important in view of the dose-dependent side-effects of acetylsalicylate. For these reasons, the composition will be effective and safe. It must be considered an additional weapon against atherosclerosis along with the reduction of known riskfactors such as hypertension, smoking, hypercholesterolemia and diabetes mellitus. The composition is designed to treat and/or prevent diseases arising from atherosclerosis such as angina pectoris, myocardial infarction, transient ischemic attacks, stroke and claudicatio intermittens.

The present invention concerns orally active dosage forms of vitamin E and acetylsalicylate for primary and secondary prevention of human atherosclerosis. With orally active dosage forms are meant tablets, capsules, pills or chewing-gum including sugar-coated tablets, film-coated tablets and enteric-coated tablets. In addition to the two active ingredients "vitamin E" and "acetylsalicylate", the dosage forms can contain inert substances (additives) such as diluents, binders, lubricants, disintegrators, coloring agents, flavoring agents and gum to enhance the physical appearance, to improve stability and to aid in disintegration after oral administration.

### REFERENCES

1) Bendich A and Machlin LJ. Safety of oral intake of vitamin E. Am J Clin Nutr 1988;48:612-9.
2) US Patent : 2,723,278. Preparation of alpha-tocopherol. Nov 8th 1955.
3) Desforges JF. The use of aspirin in ischemic heart disease. N Eng J Med 1992;327:175-81.
4) US Patent : 2,890,240. Aspirin crystallization. Jun 9th 1959.
5) Losowsky MS and Leonard PJ. Evidence of vitamin E deficiency in patients with malabsorp- tion or alcoholism and the effects of therapy. Gut 1967;8:539-43.
6) Vane RJ. Inhibition of prostaglandin synthesis as a mechanism of action for aspirin-like drugs. Nature 1971;231:232-5.
7) DeGaetano G, Cerletti C, Dejana E and Latini R. Pharmacology of platelet inhibition in humans: implications of the salicylate-aspirin interaction. Circulation 1985;72:1185-93.
8) Steinberg D, Parthasarathy S, Carew TE, Khoo JC and Witztum JL. Beyond cholesterol. Modifications of low-density lipoprotein that increase its atherogenicity. New Eng J Med;1989;320:915-24.
9) Ross R. The pathogenesis of atherosclerosis - an update. N Eng J Med 1986;314:488-500.

## Claims

1. Pharmaceutical compositions containing both vitamin E and acetylsalicylate.

2. Pharmaceutical compositions containing at least one of the following substances with vitamin E activity:
all stereoisomeric forms of alpha-tocopherol, beta-tocopherol, gamma-tocopherol, delta-tocopherol, alpha-tocotrienol, beta-tocotrienol, gamma-tocotrienol, delta-tocotrienol and their acetate- or succinate-esters;
together with :
acetylsalicylate or any salt of acetylsalicylate.

3. The pharmaceutical composition containing vitamin E and acetylsalicylate for prevention of atherosclerosis.

4. The pharmaceutical composition containing vitamin E and acetylsalicylate for treatment of atherosclerosis.

5. Orally active pharmaceutical dosage forms containing up to 2000 IU vitamin E and up to 500 mg acetylsalicylate for treatment of diseases arising from atherosclerosis.

6. The complementary beneficial action of vitamin E and acetylsalicylate on atherogenesis.
